# EUROPÄISCHE PATENTSCHRIFT

(11) **EP 1 094 763 B1**
(45) Veröffentlichungstag und Bekanntmachung des Hinweises auf die Patenterteilung: **17.11.2004**
(21) Anmeldenummer: 00936706.1
(22) Anmeldetag: 03.05.2000
(51) Int. Cl.: A61F 2/06

(54) **STENT-KATHETER-ANORDNUNG**
STENT CATHETER SYSTEM
SYSTEME TUTEUR-CATHETER

(30) Priorität: 03.05.1999 DE 29907827 U
(43) Veröffentlichungstag der Anmeldung: 02.05.2001
(73) Patentinhaber: Abbott Laboratories Vascular Enterprises Limited, Dublin 2 (IE)
(72) Erfinder: VON OEPEN, Randolf, D-72415 Hirrlingen (DE)
(74) Vertreter: Schmitz, Hans-Werner, Dipl.-Ing.
(86) Internationale Anmeldenummer: PCT/EP2000/003971
(87) Internationale Veröffentlichungsnummer: WO 2000/066033

(56) Entgegenhaltungen:
- EP-A- 0 779 062
- WO-A-96/41589
- WO-A-97/40877
- WO-A-98/02112
- DE-C- 19 509 464
- FR-A- 2 768 611

## Beschreibung

Die Erfindung betrifft eine Stent-Katheter-Anordnung nach dem Oberbegriff des Anspruchs 1.

Eine derartige Anordnung ist aus der EP 0.779 062 A1 bekannt und wird zum Beispiel dazu verwendet, Stenosen in Blutgefäßen aufzuweiten. Hierzu wird der auf dem aufweitbaren Ballon der Stent-Katheter-Anordnung angeordnete Stent in das Blutgefäß eines Patienten eingeführt und mit Hilfe eines Führungsdrahtes in den Bereich der Stenose vorgeschoben. Nach der Plazierung des Stents innerhalb der Stenose wird der Ballon durch das Einfüllen 'eines geeigneten Mediums in den Katheter aufgeweitet, so daß sich dadurch auch der Stent radial ausdehnt, bis die Stenose auf das gewünschte Maß aufgeweitet ist. Danach wird der Ballon geleert und die Katheter-Anordnung kann aus dem Körper des Patienten entnommen werden, wobei der Stent innerhalb der Stenose verbleibt und diese bleibend in der aufgeweiteten Stellung hält.

Aus der DE 195 09 464 C1 ist ein Gefäßimplantat bekannt, daß aus einem von innen an einer Gefäßwand befestigbaren Verankerungsteil und einem im Hohlraum des Gefäßes angeordneten Funktionsteil besteht. Der Funktionsteil ist dabei bis zur Längsmittelachse hin verjüngt ausgebildet und kann mit einer Beschichtung versehen werden, so daß sich dauerhafte wirksame Gefäßverschlüsse bilden.

Im Rahmen der Erfindung durchgeführte Untersuchungen haben jedoch ergeben, daß eine derartige Anordnung bei Indikationen keine Verwendung finden kann, bei denen es erforderlich ist, bewußt im Blutgefäß eines Patienten, üblicherweise bei Neugeborenen, Säuglingen, Kindern und Kleinkindern, eine Drosselung des Blutstromes zu bewirken, da die gattungsgemäße Stent-Katheter-Anordnung nur zum Gegenteil der Bewirkung einer derartigen Drosselung, nämlich zur Aufweitung eines Gefäßes und damit zur Entfernung einer Drosselung geeignet ist.

Es ist daher Aufgabe der Erfindung, eine Stent-Katheter-Anordnung der im Oberbegriff des Anspruchs 1 angegebenen Art zu schaffen, mit der es möglich ist, in Blutgefäßen eine Drosselung des Blutstromes zu bewirken.

Die Lösung dieser Aufgabe erfolgt durch die Merkmale des Anspruches 1.

Die erfindungsgemäße Stent-Katheter-Anordnung weist einen Katheter auf, der einen aufweitbaren Ballon umfaßt, der zumindest an einem seiner Bereiche, üblicherweise in etwa in seinem Mittelbereich, einen geringer aufweitbaren Abschnitt aufweist. Auf den derart ausgebildeten Ballon wird in üblicher Weise ein Stent aufgesetzt, beispielsweise aufgekrimpt, der mit einer strömungsundurchlässigen Umhüllung versehen ist.

Wird diese Anordnung in das Gefäß eines Patienten eingeführt und der Ballon aufgeweitet, ergibt sich eine im Gefäß verbleibende Ausbildung des Stents, die zwei Bereiche aufweist, mittels derer der Stent im Gefäß fixiert wird. Zwischen diesen beiden Fixierungsbereichen ergibt sich beim Aufweiten des Ballons ein Drosselabschnitt, da in diesem Bereich der Stent auf dem geringer aufweitbaren Bereich des Ballons aufliegt. Der Drosselbereich kann je nach Behandlungsfall unterschiedlich ausgebildet sein, hat aber in jedem Fall ein Volumen, das einen zwar gedrosselten, jedoch noch ausreichenden Blutfluß erlaubt.

Vorzugsweise liegt zwischen den Fixierungsbereichen und dem Drosselbereich des Stents jeweils ein sich allmählich verjüngender Bereich, so daß Verwirbelungen des Blutstromes zumindestens weitgehend vermieden werden können.

Die Unteransprüche haben vorteilhafte Weiterbildungen der Erfindung zum Inhalt.

Die Umhüllung des Stents kann mittels einer geeigneten, körperverträglichen Folie bewirkt werden, die beispielsweise aus PTFE-Material bestehen kann.

Der geringer aufweitbare Bereich des Ballons kann durch eine geeignete Versteifung des Ballonmaterials in diesem Abschnitt bewirkt werden, wozu beispielsweise im Ballonmaterial integrierte Versteifungen oder auch auf das Ballonmaterial in diesem Bereich aufgebrachte Versteifungen, wie beispielsweise ein vorzugsweise aufgeklebter Versteifungsring Verwendung finden können.

Ferner ist es möglich, den geringer aufweitbaren Bereich des Ballons im Zuge der Ballonherstellung vorzusehen. Hierzu kann die für die Ballonherstellung vorgesehene Metallform, in die das Ballonmaterial eingeblasen wird, mit einer Querschnittsverringerung an der Stelle versehen werden, an der später der geringer aufweitbare Bereich des Ballons angeordnet sein soll.

Weitere Einzelheiten, Merkmale und Vorteile der Erfindung ergeben sich aus nachfolgender Beschreibung eines Ausführungsbeispiels anhand der Zeichnung.

Es zeigt:
Fig. 1 eine perspektivische Darstellung einer erfindungsgemäßen Stent-Katheter-Anordnung mit auf einem Ballon plazierten Stent in nicht aufgeweitetem Zustand,
Fig. 2 eine der Fig. 1 entsprechende Darstellung der erfindungsgemäßen Stent-Katheter-Anordnung, bei der sowohl der Ballon als auch der Stent im aufgeweiteten Zustand dargestellt sind, und
Fig. 3 eine schematisch stark vereinfachte Darstellung eines Blutgefäßes mit in diesem angeordneten erfindungsgemäßen Stent.

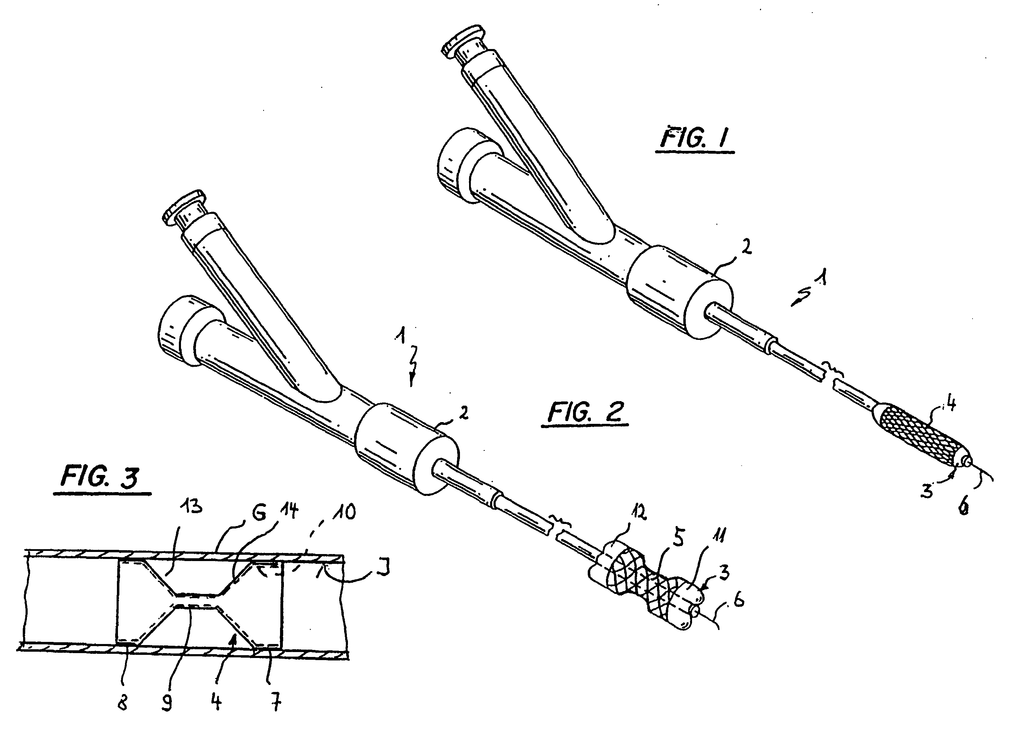

In den Fig. 1 und 2 ist eine mögliche Ausführungsform einer erfindungsgemäßen Stent-Katheter-Anordnung 1 dargestellt. Die Anordnung 1 kann einen handelsüblich ausgebildeten Katheter 2 mit einem aufweitbaren Ballon 3 aufweisen. Auf dem Ballon 3 ist bei der Darstellung gemäß Fig. 1 im nicht aufgeweiteten Zustand ein Stent 4 plaziert, beispielsweise aufgekrimpt.

Der Ballon 3 der erfindungsgemäßen Stent-Katheter-Anordnung weist einen geringer aufweitbaren Abschnitt 5 auf, der in Fig. 2 sichtbar ist, die den insgesamt aufgeweiteten Zustand des Ballons 3 darstellt. Der geringer aufweitbare Abschnitt 5 liegt zwischen zwei vollständig aufweitbaren Abschnitten 11 und 12.

In den Fig. 1 und 2 ist neben weiteren an sich bekannten Merkmalen des Katheters 2 ein Führungsdraht 6 sichtbar, mit Hilfe dessen der Stent 4 im Gefäß eines Patienten plaziert werden kann.

Fig. 3 zeigt diesen Zustand. In Fig. 3 ist in schematisch vereinfachter Darstellung ein Gefäß G eines Patienten gezeigt, in dem ein erfindungsgemäßer Stent 4 angeordnet ist. Durch das Aufweiten des Ballons 3 haben sich zwei Fixierungsabschnitte 7 und 8 ergeben, mit Hilfe derer der Stent 4 im Gefäß G, also an dessen Innenwandung I fixiert ist. Zwischen den Fixierungsbereichen 7 und 8 liegt ein Drosselbereich 9, der in seinem Querschnitt deutlich gegenüber den Fixierungsabschnitten 7 und 8 vermindert ist. Die Verminderung ergibt sich aufgrund des geringer aufweitbaren Abschnittes 5 des Ballons 3 beim Aufweiten des Ballons 3, wie dies in Fig. 2 verdeutlicht ist.

Zwischen den Fixierungsabschnitten 7 und 8 und dem Drosselabschnitt 9 liegt jeweils ein sich verjüngender Abschnitt 13 und 14.

Fig. 3 zeigt ferner in vereinfachter Darstellung eine Umhüllung (Ummantelung) 10, die die gesamte Stentstruktur, die üblicherweise eine Stegstruktur ist, bedeckt, damit die an sich blutdurchlässige Stegstruktur des erfindungsgemäßen Stents 4 flüssigkeitsundurchlässig gemacht wird.

## Patentansprüche

1. Stent-Katheter-Anordnung (1)
- mit einem Katheter (2), der einen aufweitbaren Ballon (3) mit einem ersten vollständig aufweitbaren Abschnitt (11) und einem zweiten vollständig aufweitbaren Abschnitt (12) und einem zwischen diesen angeordneten reduziert aufweitbaren Abschnitt (5) aufweist; und
- mit einem Stent (4), der auf dem aufweitbaren Ballon (3) platzierbar ist,
**dadurch gekennzeichnet,**
- **dass** der Stent (4) mit einer flüssigkeitsundurchlässigen Umhüllung (10) zur Bildung eines Drosselabschnittes (9) zwischen zwei Fixierungsabschnitten (7, 8) versehen ist, wobei der Drosselabschnitt (9) des Stents (4) im in einem Gefäß (G) implantierten Zustand einen Abstand zu der Innenwandung (I) des Gefäßes (G) einnimmt.

2. Stent-Katheter-Anordnung nach Anspruch 1, **dadurch gekennzeichnet, daß** die Umhüllung (10) eine Folie oder ein Mantel ist.

3. Stent-Katheter-Anordnung nach Anspruch 2, **dadurch gekennzeichnet, daß** die Folie beziehungsweise der Mantel aus körperverträglichem Material besteht.

4. Stent-Katheter-Anordnung nach Anspruch 2 oder 3, **dadurch gekennzeichnet, daß** die Folie beziehungsweise der Mantel aus biologischem Material, aus Polymer-Material, aus metallischem Material, keramischem Material oder Elastomer-Material besteht.

5. Stent-Katheter-Anordnung nach einem der Ansprüche 1 bis 3, **dadurch gekennzeichnet, daß** der reduziert aufweitbare Abschnitt (5) des Ballons (3) aus versteiftem Ballon-Material besteht.

6. Stent-Katheter-Anordnung nach einem der Ansprüche 1 bis 3, **dadurch gekennzeichnet, daß** der reduziert aufweitbare Abschnitt (5) des Ballons (3) durch ein auf dem aufweitbaren Material des Ballons aufgebrachtes Versteifungselement gebildet ist.

7. Stent-Katheter-Anordnung nach einem der Ansprüche 1 bis 5, **dadurch gekennzeichnet, daß** der reduziert aufweitbare Abschnitt (5) des Ballons (3) im Zuge der Ballonherstellung erzeugbar ist.

## Claims

1. Stent-catheter arrangement (1)
- having a catheter (2) comprising an expandable balloon (3) having a first completely expandable section (11) and a second completely expandable section (12) and a reducedly expandable section (5) arranged between these; and
- having a stent (4) which is placeable on the expandable balloon (3),
**characterised in that**
- the stent (4) is provided with a liquid-impermeable sheathing (10) for forming a constricted section (9) between two fixing sections (7, 8), wherein the constricted section (9) of the slant (4) in the implanted state in a vessel (G) assumes a spacing relative to the inner wall (I) of the vessel (G).

2. Stent-catheter arrangement according to claim 1, **characterised in that** the sheathing (10) is a film or a mantle.

3. Stent-catheter arrangement according to claim 2, **characterised in that** the film or the mantle is composed of biocompatible material.

4. Stent-catheter arrangement according to claim 2 or 3, **characterised in that** the film or the mantle is composed of biological material, of polymeric material, of metallic material, ceramic material or elastomeric material.

5. Stent-catheter arrangement according to one of claims 1 to 3, **characterised in that** the reducedly expandable section (5) of the balloon (3) is composed of stiffened balloon material.

6. Stent-catheter arrangement according to one of claims 1 to 3, **characterised in that** the reducedly expandable section (5) of the balloon (3) is formed by a stiffening element applied to the expandable material of the balloon.

7. Stent-catheter arrangement according to one of claims 1 to 5, **characterised in that** the reducedly expandable section (5) of the balloon (3) is producible in the course of balloon manufacture.

## Revendications

1. Dispositif de cathéter avec stent (1)
- avec un cathéter (2) qui comporte un ballon gonflable (3) avec une première portion (11) complètement gonflable et une seconde portion (12) complètement gonflable et une portion (5) moins gonflable agencée entre ces deux portions ; et
- avec un stent (4) qui peut être placé sur le ballon gonflable (3), **caractérisé en ce que**,
- le stent (4) est pourvu d'une gaine (10) imperméable aux liquides pour former une portion d'étranglement (9) entre deux portions de fixation (7, 8), la portion d'étranglement (9) du stent (4) se plaçant, à l'état implanté dans un vaisseau (G), à distance de la paroi intérieure (I) du vaisseau (G).

2. Dispositif de cathéter avec stent selon la revendication 1, **caractérisé en ce que** la gaine (10) est une feuille ou une enveloppe.

3. Dispositif de cathéter avec stent selon la revendication 2, **caractérisé en ce que** la feuille ou l'enveloppe est constituée d'une matière compatible avec le corps.

4. Dispositif de cathéter avec stent selon la revendication 2 ou 3, **caractérisé en ce que** la feuille ou l'enveloppe est constituée d'une matière biologique, d'une matière polymère, d'une matière métallique, d'une matière céramique ou d'une matière élastomère.

5. Dispositif de cathéter avec stent selon l'une quelconque des revendications 1 à 3, **caractérisé en ce que** la portion (5) moins gonflable du ballon (3) est constituée d'une matière de ballon renforcée.

6. Dispositif de cathéter avec stent selon l'une quelconque des revendications 1 à 3, **caractérisé en ce que** la portion (5) moins gonflable du ballon (3) est formée par un élément de renforcement monté sur la matière gonflable du ballon.

7. Dispositif de cathéter avec stent selon l'une quelconque des revendications 1 à 5, **caractérisé en ce que** la portion (5) moins gonflable du ballon (3) peut être créée pendant la fabrication du ballon.
